(19)

Europäisches
Patentamt

European
Patent Office

Office européen
des brevets

(11) **EP 2 674 886 A2**

(12) **EUROPEAN PATENT APPLICATION**

(43) Date of publication:
**18.12.2013 Bulletin 2013/51**

(51) Int Cl.:
***G06F 19/24*** *(2011.01)*      *G06F 19/26* *(2011.01)*

(21) Application number: **13172115.1**

(22) Date of filing: **14.06.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.06.2012 US 201261660581 P**
**13.03.2013 US 201313799139**

(71) Applicant: **Thermo Fisher Scientific Oy**
**01620 Vantaa (FI)**

(72) Inventors:
• **Mustola, Jorma**
**09430 SAUKKOLA (FI)**

• **Kurkela, Jaakko**
**02780 ESPOO (FI)**
• **Vahervuori, Ville**
**01640 VANTAA (FI)**
• **Saris, Ossian**
**02610 ESPOO (FI)**
• **Kavanagh, Ian**
**6006 LUZERN (CH)**
• **Andre, Charles**
**SAN FRANCISCO, CA California 94114 (US)**
• **Cohen, David**
**ESPOO 02230 (FI)**

(74) Representative: **Berggren Oy Ab**
**P.O. Box 16**
**Antinkatu 3 C**
**00101 Helsinki (FI)**

(54) **Methods and systems for high resolution melt analysis of a nucleic acid sequence**

(57)      Described herein are methods and systems for analyzing and visualizing HRM data from a double-stranded nucleic acid. The HRM data is generally characterized by a plurality of data points each including a signal value associated with the concentration of a double-stranded nucleic acid in a sample and a temperature value associated with a the temperature of the sample. Embodiments of the invention analyze the HRM curves from samples using the first negative derivative of the HRM curve or a virtual standard. The first negative derivative plot method may be used to identify the melting temperature of a homogenous double-stranded nucleic acid in a sample, as well as the presence and melting temperature of heterogeneous double-stranded nucleic acids in the sample. Data points associated with the melting temperature are plotted on a scatter plot for analysis. The virtual standard allows for visualization of HRM data across data sets.

FIG. 2

**EP 2 674 886 A2**

**Description**

**CROSS-REFERENCE TO RELATED APPLICATIONS**

This application claims the benefit of and priority to prior filed pending Provisional Application Serial No. 61/660,581, filed June 15, 2012 and to prior filed pending Utility Application Serial No. 13/799,139, filed March 13, 2013, which are expressly incorporated herein by reference.

**FIELD**

The present invention relates generally to the analysis of double-stranded nucleic acids and, more particularly, to the high resolution melt analysis of double-stranded nucleic acids.

**BACKGROUND**

High resolution melt (HRM) analysis allows for the detection of mutations, polymorphisms, and epigenetic differences in double-stranded nucleic acids in a sample without sequencing the nucleic acids. Typically, for HRM analysis, a target nucleic acid sequence is amplified using the polymerase chain reaction (PCR) technique in the presence of a reporter molecule, such as a fluorescent dye, that selectively fluoresces when associated with a double-stranded nucleic acid. It has been observed that the signal produced from monitoring the slow melt of a double-stranded nucleic acid, such as an amplified DNA sequence, follows a generally sigmoidal pattern in which the signal level decreases as a function of temperature. The shape of the HRM curve and the melt temperature, i.e., the temperature at which the signal exhibits the greatest amount of change, is determined by the specific sequence of nucleotides composing double-stranded nucleic acid. Samples having mixtures of double-stranded nucleic acids, such as occurs with a heterogeneous sample, or samples having one or more mutations, will exhibit changes in the shape of the HRM curve and/or a shift in the melting temperature.

Existing HRM analysis is done by use of difference plot visualization, in which the changes in signal level of a data set from a first sample is used as a baseline, and the difference from data sets from other samples to the baseline is plotted across the entire temperature range. While this method can allow for the grouping of similar double-stranded nucleic acids, the results can be difficult to analyze, the view of the results is dependent upon the data chosen as the baseline, and automated grouping algorithms can be difficult to construct or can be confused by the baseline choice thus leading to different results. A need for a better method of analyzing HRM data was thus identified.

**BRIEF SUMMARY**

Before HRM analysis was available, the temperature resolution of instruments was not able to distinguish small changes in double-stranded nucleic acid melt temperature such as those caused by single nucleotide changes. Even after HRM capable instruments have become available, melt temperature information has not been considered informative enough as melt temperature of a heterozygote sample may be very close to homozygote melt temperature. However, the when melt temperature data is combined with other information such as at least one of a peak height value, a peak width value, or an area under the curve value, discrimination between the melt temperatures from double stranded nucleic acids is greatly enhanced.

Described herein are methods and systems for analyzing and visualizing HRM data from a double-stranded nucleic acid utilizing data points associated with the HRM data that includes a combination of the melt temperature and at least one of a peak height value, a peak width value and/or an area under the curve value. The HRM data is generally characterized by a plurality of data points each including a signal value associated with the concentration of a double-stranded nucleic acid in a sample and a temperature value associated with a the temperature of the sample. Embodiments of the invention analyze the HRM curves of samples using the first negative derivative of the HRM curve or by difference plot visualization of the HRM data using a virtual standard.

In one embodiment, the method includes generating a HRM curve from the HRM data for each sample and plotting the first negative derivative of the HRM curves. The melt peak for each sample is identified from the first negative derivative plot for each sample and analyzed. In an embodiment, the melt peak, which represents the melt temperature for the sample, is identified as the data point along the first negative derivative plot having the greatest distance from the x-axis.

In an alternative embodiment, a Gaussian probability function is fit to the first negative derivative plot and the melt peak is the data point along the Gaussian probability function having the greatest distance from the x-axis.

In another alternative embodiment, the Gaussian probability function is subtracted from the first negative derivative curve and a second melt peak from the subtracted data set is identified. Additional melt peaks can be identified with additional Gaussian probability subtraction steps.

In another alternative embodiment, HRM curves from at least two samples are normalized relative to one another. The first negative derivative is then plotted for the normalized HRM curves for each sample and the melt peaks for each sample are identified and analyzed.

In an alternative embodiment, the melt peak is identified for a first negative derivative plot and the width of the plot is calculated at a fraction of the melt peak height. A data point having a temperature value and at least one of a width value or a peak height value is then analyzed, such as by plotting on a scatter plot.

Another aspect of the invention is directed to improved methods of visualizing HRM data for one or more samples by generating a HRM curve for each sample, providing a virtual standard, and plotting the differences between the HRM curve for each sample and the virtual curve. An alternative embodiment further includes plotting the first negative derivative of the HRM curve and the virtual standard and then plotting the differences between the first negative plot for each sample and the virtual curve.

The virtual curve can be provided using a number of techniques. In one embodiment, the signal values for the virtual curve are derived from the averages of the signal values for the HRM curves from the samples. In another embodiment, the virtual curve is a derived from the theoretical melting profile of a target double-stranded nucleic acid. In yet another embodiment, the virtual curve is calculated from a formula with variables that may be adjusted by the user. In a further embodiment, the virtual standard includes a spline curve in a sigmoidal shape that may be altered by the user using a computer interface.

## BRIEF DESCRIPTION OF SEVERAL VIEWS OF THE DRAWINGS

The accompanying drawings, which are incorporated in and constitute a part of this specification, illustrate various embodiments of the invention and, together with a general description of the invention given above and the detailed description of the embodiments given below, serve to explain the embodiments of the invention.

FIG. 1A is a graph illustrating an exemplary HRM curve in accordance with embodiments of the invention.

FIG. 1B is a graph illustrating an exemplary plot of the first negative derivative of the HRM curve of FIG. 1A in accordance with embodiments of the invention.

FIG. 1C is a graph illustrating a first and a second Gaussian distribution curve fit to the exemplary plot of the first negative derivative of a the HRM curve of FIG. 1B in accordance with embodiments of the invention.

FIG. 1D is a scatter plot of data points derived from a first negative derivative plot in accordance with embodiments of the invention.

FIG. 2 is a flow chart illustrating a process of analyzing HRM data in accordance with embodiments of the invention.

FIG. 3 is a flow chart illustrating a process for generating a HRM curve in accordance with embodiments of the invention.

FIG. 4 is a flow chart illustrating a process of analyzing HRM data in accordance with embodiments of the invention.

FIG. 5 is a flow chart illustrating a process of analyzing HRM data in accordance with embodiments of the invention.

FIG. 6 is a flow chart illustrating a process of analyzing HRM data in accordance with embodiments of the invention.

FIG. 7 is a flow chart illustrating a process of analyzing HRM data in accordance with embodiments of the invention.

FIG. 8A is a graph illustrating a process for visualizing HRM data in accordance with embodiments of the invention.

FIG. 8B is a graph illustrating a process for visualizing HRM data on a difference plot in accordance with embodiments of the invention.

FIG. 9 is a flow chart illustrating a process for visualizing HRM data in accordance with embodiments of the invention.

FIG. 10 is a flow chart illustrating a process for visualizing HRM data in accordance with embodiments of the invention.

FIG. 11 is a flow chart illustrating a process for generating a virtual standard in accordance with embodiments of the invention.

FIG. 12 is a flow chart illustrating a process for generating a virtual standard in accordance with embodiments of the invention.

FIG. 13 is a block diagram of a computer system in accordance with embodiments of the invention.

FIG. 14A is a graph illustrating HRM curves with the exponential decay removed in accordance with embodiments of the invention.

FIG. 14B is a graph illustrating the HRM curves of FIG. 14A after being normalized in accordance with embodiments of the invention.

FIG. 14C is a graph illustrating a plot of the first negative derivative of the normalized HRM curves of FIG. 14B in accordance with embodiments of the invention.

FIG. 14D is a scatter plot of data points derived from the graph of FIG. 14C in accordance with embodiments of the invention.

FIG. 15A is a graph illustrating a plot of the first negative derivative of the HRM curves from a homogenous and heterogeneous double-stranded nucleic acid in accordance with embodiments of the invention.

FIG. 15B is a scatter plot of melt peak data points derived from the graph of FIG. 15A in accordance with embodiments of the invention.

FIG. 15C is a scatter plot of peak width data points derived from the graph of FIG. 15A in accordance with embodiments of the invention.

## DETAILED DESCRIPTION

Complimentary strands of nucleic acids form relatively stable double strands of nucleic acids at lower temperatures. As the temperature of a sample containing a double-stranded nucleic acid is increased, the double-stranded nucleic acid melts into two single strands. Similarly, as the temperature of a sample containing complimentary single strands of nucleic acid is decreased, the complimentary nucleic acids will reassociate into double-stranded nucleic acids. The melt temperature of a double-stranded nucleic acid, i.e., the temperature at which a nucleic acid transitions between a double-stranded nucleic acid and a pair of single strands, is determined by the length and sequence of the nucleic acid strands. Differences between two or more double-stranded nucleic acids, such as double-stranded nucleic acids amplified using polymerase chain reaction (PCR), may be inferred by observing and analyzing the high resolution melting or high resolution reassociation of the double-stranded nucleic acids over a range of temperatures. As used herein, the terms "high resolution melting" or "high resolution melt" are understood to include both high resolution melt and high resolution reassociation.

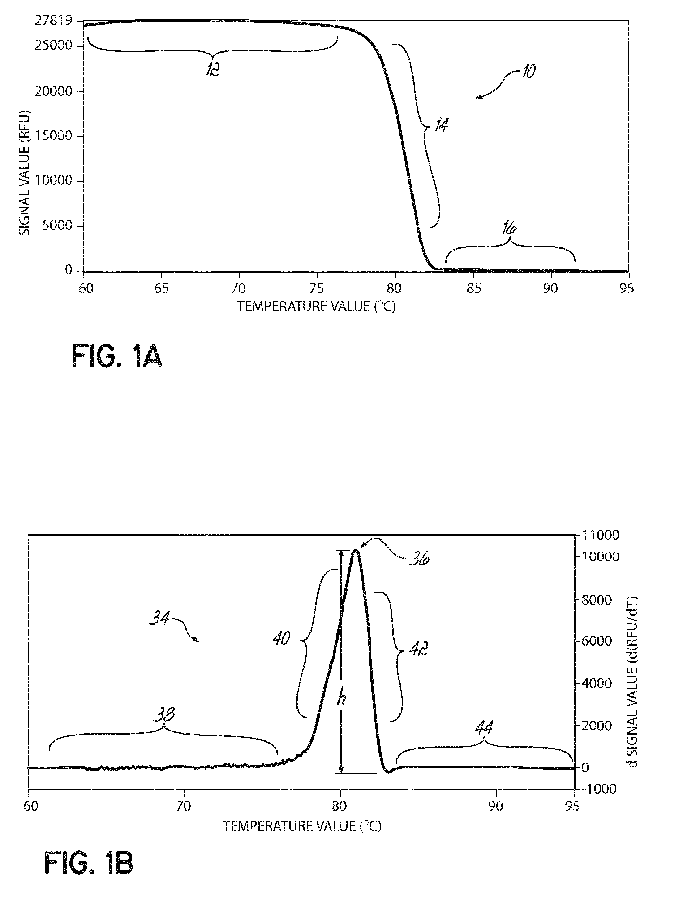

With reference to FIG. 1A, and in accordance with embodiments of the invention, an improved method of analyzing high resolution melt (HRM) data includes analyzing data collected from the high resolution melting of a sample that includes at least one amplified double-stranded nucleic acid wherein the data is characterized by an ascending HRM curve 10. Similarly, the HRM data may be collected from the high resolution reassociation of a sample that includes complimentary single strands of a double-stranded nucleic acid. The HRM data from high resolution reassociation data is characterized by a descending HRM curve (not shown). The HRM curve 10 includes a temperature value along the x-axis and a signal value representative of the concentration of double-stranded nucleic acid in the sample at a given temperature value along the y-axis. In an exemplary embodiment, the temperature value is expressed in degrees Celsius and the signal value is expressed as relative fluorescent units (RFU's).

In an exemplary embodiment, the signal value for a sample is obtained with a reporter molecule that selectively fluoresces when associated with a doubled stranded nucleic acid. Thus, the signal value, i.e., the level of fluorescence observed in a sample, is indicative of the concentration of double-stranded nucleic acid in the sample. Reporter molecules useful with embodiments of the invention described herein are those that selectively provide a signal, such as a fluorescent signal, when associated with a double-stranded nucleic acid. For example, fluorescent double-stranded nucleic dyes used in real time PCR reactions may be used. Exemplary reporter molecules include SYBR® Green I, SYBR® Gold, PicoGreen ® (each available from Invitrogen), and LC Green®, Eva Green, Melt Doctor, SYTO®-9, SYTO®-13, SYTO®-16, SYTO®-60, SYTO®-62, SYTO®-64, SYTO®-82, POPO-3, TOTO-3, PO-PRO-3, TO-PRO-3, YO-PRO®-1, SYTOX® Orange, BEBO, BOXTO, Chromofy, as well as other reporter molecules that selectively fluoresce when associated with double-stranded nucleic acids. In addition to the use of reporter molecules that selectively associate with double stranded nucleic acids, the reporter molecules may also be associated with fluorescent probes or primer based systems. As used herein, the term reporter molecule is understood to include any system, molecule, probe, dye, or combination thereof that is capable of generating a signal that corresponds to the concentration of double-stranded nucleic acid in a sample at a particular temperature.

For high resolution melting, the signal value is obtained from measurements taken at predetermined increments as the temperature of the sample is slowly increased from a temperature at which substantially all of the complementary nucleic acid strands in the sample are in the double-stranded state, to a temperature at which no double-stranded nucleic acid is detectable with the reporter molecule. For high resolution reassociation, the signal value is obtained from measurements taken at predetermined increments as the temperature of the sample is slowly decreased from a temperature at which substantially all of the complementary nucleic acid strands in the sample are in the single-stranded state, to a temperature at which substantially all of the nucleic acid is in a double-stranded state as detected with the reporter molecule. Typically, the signal value is measured over a range of temperatures from about 60 degrees Celsius to about 95 degrees Celsius; however, the temperature range may be increased or decreased as needed to analyze a specific nucleic acid sequence.

In accordance with embodiments of the invention, the signal value is obtained as the temperature increases by fractions of a degree over at least a portion of the melting temperature range. In an embodiment, the signal value is obtained at about every 0.1 degrees Celsius over at least a portion of the melting temperature range. In an alternative embodiment, the signal value is obtained at about every 0.2 degrees Celsius over at least a portion of the melting temperature range. In an alternative embodiment, the signal value may be obtained at about every 0.04 degrees Celsius to about 5.0 degrees Celsius over at least a portion of the melting temperature range.

With reference to FIG. 1A, the data obtained from HRM analysis of a double-stranded nucleic acid generally forms a sigmoid shaped curve 10 having a saturation region 12, a region of exponential melting 14, and a background region 16. The saturation region 12 is a relatively flat region typically at lower temperature values in the curve 10 and characterized by high signal values because the double-stranded nucleic acid concentration in the sample is constant because at these temperatures the concentration of double-stranded nucleic acid in the sample does not change thus there is no change in the signal generated by the reporter molecule. In some embodiments, the double-stranded nucleic acid is saturated with reporter molecules whereas in other embodiments, the double-stranded nucleic acid is not saturated. The region of exponential melting 14 is the central region of the curve 10 wherein the signal value generated by the reporter molecule changes in relation to exponential changes in the concentration of double-stranded nucleic acid as the double-stranded nucleic acid melts or reassociates with changes in the temperature. The background region 16 is a relatively flat region typically at higher temperature values. In the background region 16, the concentration of double-stranded nucleic acids in the sample is reduced to levels that the signal from the reporter molecule associated with double-stranded nucleic acids cannot overcome the background signals in the reaction chamber.

HRM curves, such as shown in FIG. 1A, are generally analyzed to determine if two or more samples include identical or different double-stranded nucleic acid sequences. To make this determination, HRM curves from two or more samples are typically analyzed for differences between the curves from the samples. Differences between HRM curves generated from the samples may be observed as differences in the melt temperature of the double-stranded nucleic acids from the samples, differences in the shape of the HRM curves, or differences in both the melt temperatures and the shapes of the HRM curves. The melt temperature is the temperature at which the greatest amount of double-stranded nucleic acid melts. For HRM curve analysis, the melt temperature is the temperature at which the absolute value of the slope in the region of exponential melting 14 is the greatest.

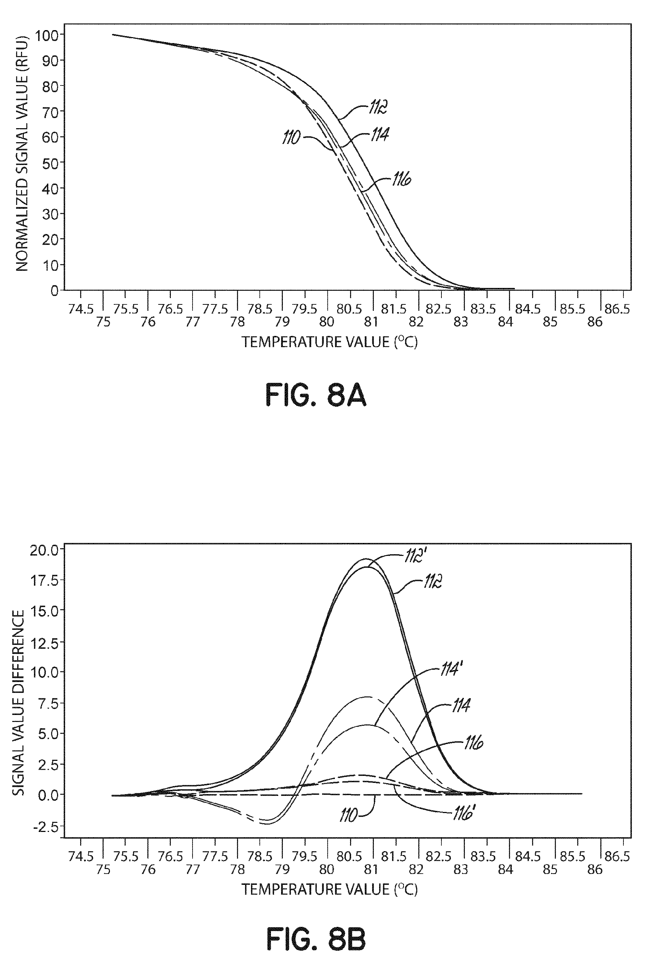

In contrast to routine methods of analyzing HRM data, embodiments of the invention analyze the HRM curves of samples using a plot of the first negative derivative of the HRM curves or the visualization of HRM data using a virtual standard. Embodiments of these methods are referred to herein as the "derivative plot methods" of FIGS. 1A-7 and the "virtual standard methods" of FIGS. 8A-12.

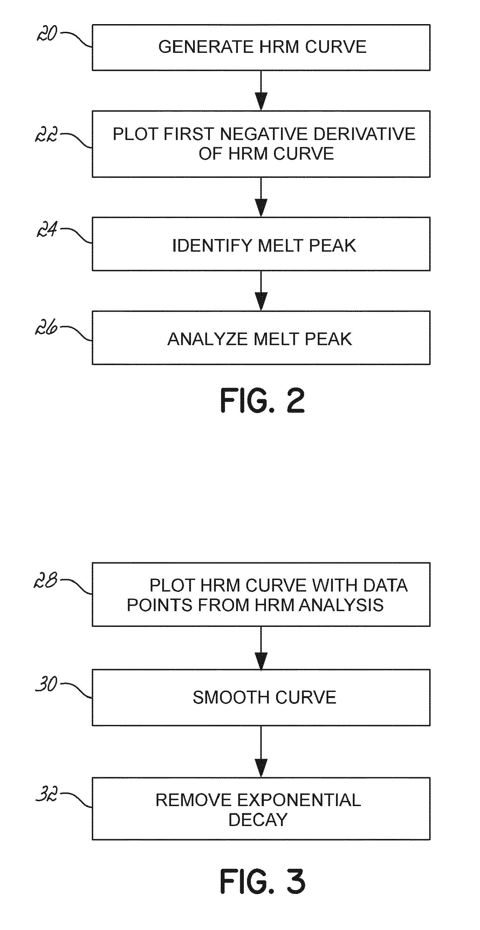

With reference to FIG. 2, the derivative plot methods generally include the generation of a HRM curve (block 20), plotting the first negative derivative of the HRM curve (block 22), identifying a melt peak of the first negative derivative plot (block 24), and analyzing the melt peak (26).

FIG. 1A illustrates an exemplary HRM curve 10. FIG. 3 illustrates an exemplary method of generating an HRM curve for analysis in accordance with embodiments of the invention. First, a HRM curve is plotted utilizing HRM data gather from the high resolution melting or high resolution reassociation of a sample containing a double-stranded nucleic acid and a reporter molecule (block 28). The HRM data includes a series of data points having a signal value and a temperature value. The HRM data may be obtained from an HRM analysis system, such as one that includes a thermal cycler for heating and/or cooling the sample in a controlled manner and an optical system for obtaining signal values as the sample is heated or cooled.

With continued reference to FIG. 3, the HRM data may optionally be internally smoothed (block 30), have the exponential decay removed (block 32), or may be both internally smoothed (block 30) and have the exponential decay removed (block 32).

The internal smoothing process (block 30) may employ any process that internally removes insignificant variations in the data that are not associated with changes in the concentration of double-stranded nucleic acid. For example, in one embodiment, the smoothing process employs a rolling average method that averages the product values for a plurality of consecutive data points from the HRM data. In another embodiment, the data are smoothed with a Savitzky-Golay smoothing filter by fitting an $n^{th}$ degree polynome to a plurality of consecutive data points and calculating a smoothed product value for one or several data points with the plurality of data points. In one embodiment, the user may optionally designate the number of data points used for the rolling average.

The exponential decay removal (block 32) process removes decreasing signal value trends that are not related to changes in the double-stranded nucleic acid concentration. Exponential decay can be removed by known processes, such as mathematical processes that calculate the amount of decay observed in the saturation region 12 (FIG. 1A). For example, a line segment may be fit by linear regression to a subset of data points in the saturation region. The slope of the line segment may then be used to correct the HRM curve 10. In another example, the exponential decay is removed from the curve directly by multiplying the measured melting curve by a correction function which is exponentially dependent on the temperature.

After generating the HRM curve, the first negative derivative is plotted for the HRM curve (block 32 of FIG. 2) and the first negative derivative plot is analyzed to identify its melt peak (block 46).

FIG. 1B an exemplary first negative derivative plot 34. The first negative derivative plot 34 of a homogenous sample, i.e., a sample that mostly contains complimentary double-stranded nucleic acids, has five regions: (1) a first background region 38, (2) a ascending region 40, (3) a melt peak 36, and (4) a descending region 42, and (5) a background region 44. The first negative derivative curve of a heterogeneous sample, i.e., a sample that includes two or more different nucleic acids, will have two or more melt peaks associate with the two or more nucleic acids, but the two or more melt peaks may mask one another such that the first negative derivative curve of the heterogeneous sample also appears to have the same five regions. The first and second background regions 38, 44 of the first negative derivative plot 34 of FIG. 1B correspond to the saturation region 12 and background region 16, respectively, of the HRM curve 10 of FIG. 1A. The ascending region 40 and the descending region 42 of the first negative derivative plot 34 of FIG. 1B correspond to portions of the exponential melting region 14 of the HRM curve 10 of FIG. 1A. The melt peak 36 of the first negative derivative plot 34 of FIG. 1B corresponds to the point along the exponential melting region 14 of FIG. 1A having the steepest slope, i.e., the melt temperature.

As illustrated in FIG. 1B, in an embodiment, the melt peak 36 is represented in the first negative derivative plot 34 as the data point having the greatest amplitude, i.e., the greatest distance h from the x-axis where the x-axis value is zero. The melt peak has a height value that corresponds to the first negative derivative of the signal value and a temperature value that corresponds with the melt temperature for the sample. The melt peaks from one or more samples may then be analyzed (block 26 of FIG. 2), as discussed in greater detail below.

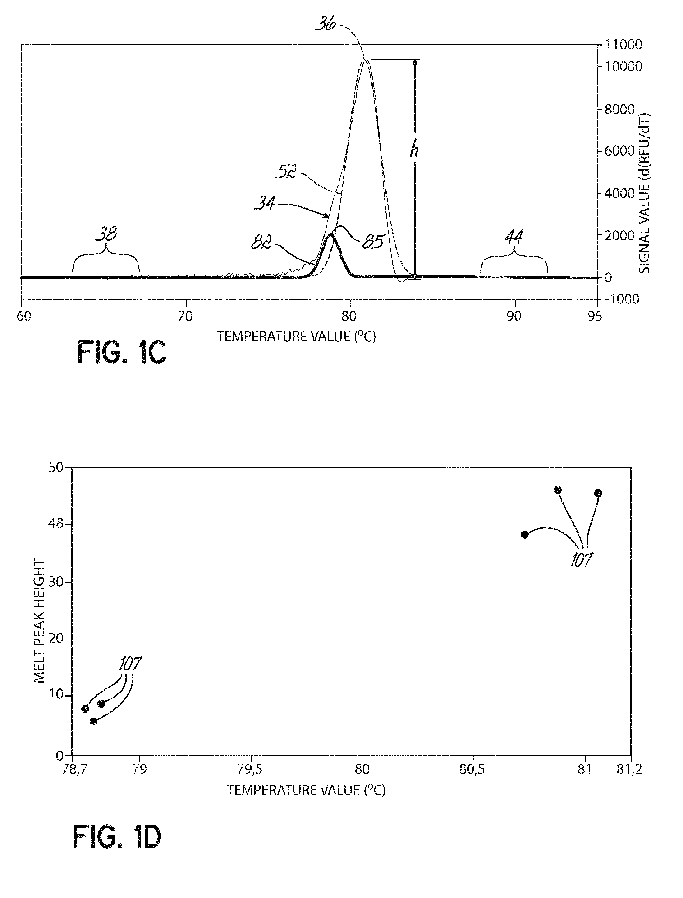

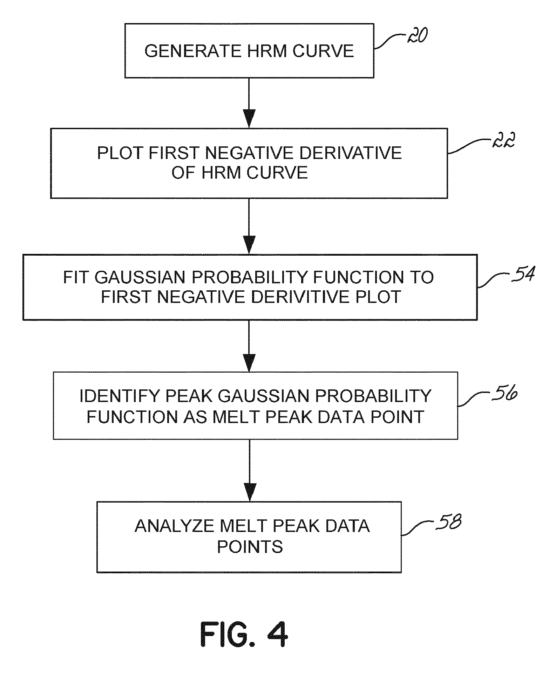

In an alternative embodiment, illustrated in FIGS. 1C and 4, the melt peak 36 is identified from a Gaussian probability function 52 that is fit to the first negative derivative curve 34. A HRM curve is generated with HRM data from a sample (block 20) and the first negative derivative of the HRM curve is plotted (block 22). A Gaussian probability function 52 is then fit to the first negative derivative curve 34 (block 54). The peak of the first Gaussian probability function 52 is identified as the melt peak 36, i.e., the point along the function 52 having the greatest amplitude, i.e., the greatest distance h from the x-axis where the x-axis value is zero (block 56). The melt peak data point may then be analyzed (block 58), as discussed in greater detail below.

The melt peak data point from a first sample may be compared with the melt peak(s) of one or more other samples, or compared to known standard values, to determine if the sequences of the samples are the same or different. If the melt peaks from the one or samples are different from one another, i.e., have a different signal value, a different temperature value, or both the signal value and the temperature value are different, then the sequences of the nucleic acids in the samples are not identical. In contrast, if the melt peaks from the sample are the same, then the sequences of the nucleic acids in the samples are likely to be the same. Processes for analyzing the melt peaks are discussed in greater detail below.

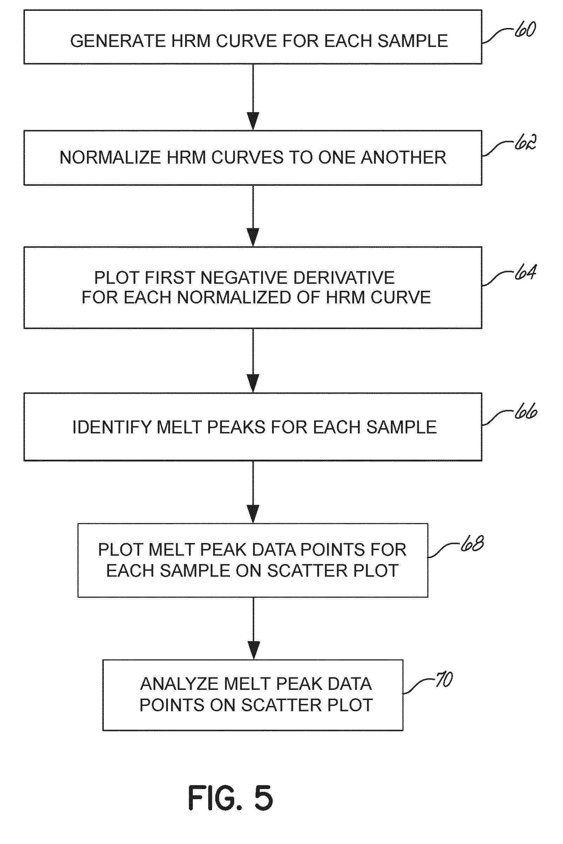

Comparing the peak values between samples can be difficult due to variability in the peak values that is not associated with the double-stranded nucleic acids in the samples. For example, the melt peak values can vary due to the position of the reaction well on the thermal block or due to inaccuracies in measuring the reagents used in the analysis. In an embodiment illustrated in FIG. 5, the HRM curves from a plurality of samples are normalized before plotting the first negative derivative for each HRM curve to account for at least a portion of the variability in the melt peaks that is not associated with the double-stranded nucleic acids in the samples. Normalizing the HRM curves results in first negative derivative plots that have identical areas under the curves. Because the area under the curve for the normalized plots are identical, any significant difference in the melt peaks for each plot indicates a difference in the shapes of the underlying HRM curves and, correspondingly, indicates a difference in the double-stranded nucleic acids from which the HRM curves originate. For this embodiment, HRM curves are generated from the HRM data for each sample (block 60). The HRM curves may optionally be internally smoothed and the exponential decay removed as previously described. The HRM curves are then normalized relative to one another (block 62, as discussed below). The first negative derivative is plotted for each of the normalized HRM curves (block 64) and the melt peaks are identified for the first negative derivative plot from the normalized HRM curves for each sample (block 66). The melt peaks are plotted as data points on a scatter plot (block 68). The melts peaks for each sample may then be analyzed to determine if they are the same or different (block 70). This method has the advantage of distinguishing melt peaks that have very similar melt temperatures, but different HRM curve shapes. In a variation of this embodiment, a Gaussian probability function is fit to the first negative derivative plot and the melt peak is identified as the peak of the Gaussian probability function, as described above with reference to FIG. 4.

HRM data may be normalized by any process that normalizes the data along the thermal axis (x-axis), the signal axis (y-axis) or along both the thermal axis and the signal axis. For thermal axis normalization, each HRM curve is shifted on the thermal axis based its location on the thermal block as determined by the thermal characteristics of the thermal block. For example, the detected melt temperature for each well may be multiplied by a standard adjustment multiplier that corresponds to the typical variation of that well from the mean of the block. The signal axis may be normalized based on user defined areas of interest in the saturation region and the background regions or preliminary areas of interest in these regions may be automatically calculated. In one embodiment, the areas of interest are identified from a first negative derivative plot of the HRM curve. The areas of interest are the areas of the first negative derivative plot having low values that correspond to areas of the HRM curves wherein the change in slope is small. The same area of interest is used for all curves being normalized to one another. The average signal value in the areas of interest across all curves being normalized are averaged and set to a first normalized signal value, such as 100, for the area of interest associated with the saturation region, and a second normalized value, such as 0, for the area of interest associated with the background region. The remaining data points are normalized to relative to the first normalized signal value and the second normalized signal value.

In addition to providing insights into the similarities and differences of HRM curves generated from different samples, the first negative derivative plot from a sample may be analyzed to identify the presence of two or more peaks in the sample, indicating that the sample includes a heterogeneous mixture of double-stranded nucleic acids.

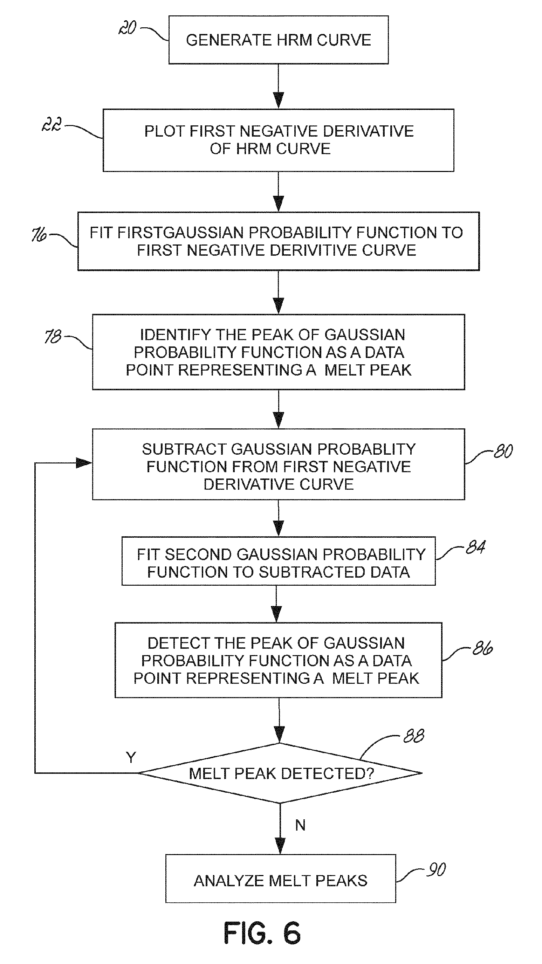

FIGS. 1C and 6 illustrates a method in accordance with embodiments of the invention to identify two or more melt peaks indicative of two or more double-stranded nucleic acid products in the sample, such as occurs with PCR reactions using primers that amplify multiple gene products. First, a HRM curve is generated (block 20), as discussed above and a first negative derivative of the HRM curve is plotted (block 22). A first Gaussian probability function 52 is fit to the first negative derivative plot (block 76). The peak of the Gaussian probability function is identified as the melt peak data point (block 78). The first Gaussian probability function is subtracted from the first negative derivative plot 34 (block 80) and a second Gaussian probability function 82 is fit (block 84) to subtracted curve. In one embodiment, the second Gaussian probability function 82 has a second melt peak 85 if the peak of the second Gaussian probability function 82 is significantly different from the first and second background regions 38, 40 (block 86), and the process is repeated until no further peaks are detected (block 88). In another embodiment, the second Gaussian probability function 82 has a second melt peak 85 if the peak of the second Gaussian probability function 82 is greater than a threshold that is calculated based on the saturation and background regions 38, 40. In an embodiment, up to four melt peaks may be identified with this process. The melt peaks are then analyzed by plotting on a scatter plot (block 90).

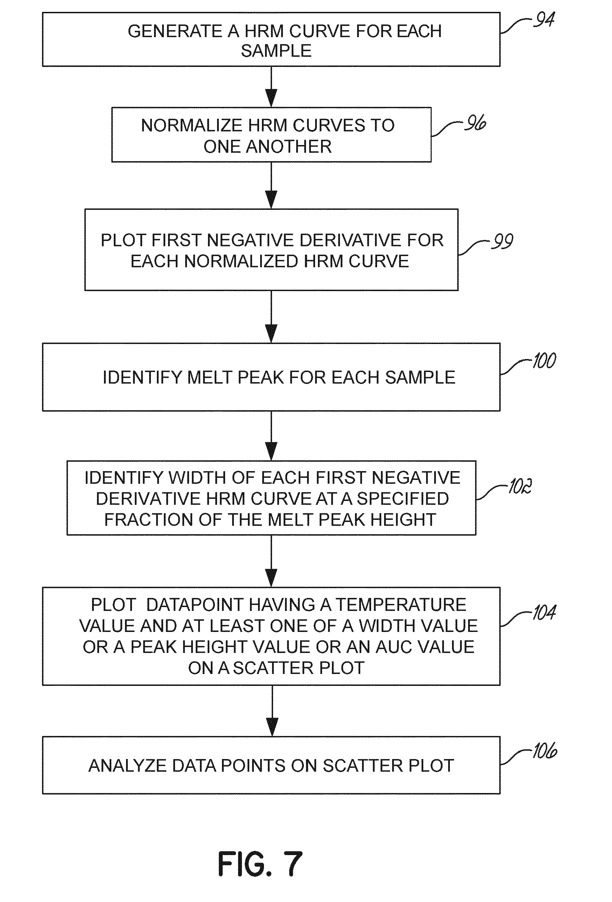

In another embodiment illustrated in FIG. 7, the width of the first negative derivative plot at a fraction of the peak height is calculated and used to distinguish HRM curves between samples. For example, a HRM curve is generated from HRM data for each sample (block 94). The HRM curve 10 may be smoothed and the exponential decay removed as shown in FIG. 1A. The HRM curves are normalized to one another (block 96) and the first negative derivative is plotted for the normalized HRM curves for each sample (block 98). The melt peak is identified for each sample as discussed above (block 100). The width of the first negative derivative plot at a specified fraction of the melt peak height is calculated or measured (block 102). In one embodiment, the width is determined at about fifty percent of the melt peak height. In an alternative embodiment, the width is determined at an optimum fraction of the melt peak height that is selected from the range between about 15 percent and about 85 percent of the melt peak height. The same fraction of the melt peak height is used to calculate or measure the width of the HRM curves across all samples. In one embodiment, the width of the first negative derivative plot and the melt temperature, i.e., the temperature value from the melt peak, is plotted on a two dimensional scatter plot (block 104). In an alternative embodiment, the signal value from the melt peak is plotted along with the width value and the temperature value in a three dimensional scatter plot (block 104). The data points on the scatter plots are then analyzed (block 106) as discussed below.

In another embodiment, also illustrated in FIG. 7, the area under the curve (AUC) of the first negative derivative plot is calculated at a fraction of the peak height and used to distinguish HRM curves between samples. For example, a HRM curve is generated from HRM data for each sample. The HRM curves may be smoothed and the exponential decay removed (not shown). The HRM curves are normalized to one another (block 96) and the first negative derivative is plotted for the normalized HRM curves for each sample (block 98). The melt peak is identified for each sample as discussed above (block 100). The AUC of the first negative derivative plot is calculated or measured (block 102) at a specified fraction of the melt peak height. In one embodiment, the AUC is determined at about fifty percent of the melt peak height. In an alternative embodiment, the AUC is determined at an optimum fraction of the melt peak height that is selected from the range between about 15 percent and about 85 percent of the melt peak height. The same fraction of the melt peak height is used to calculate or measure the AUC of the HRM curves across all samples. In one embodiment, the AUC of the first negative derivative plot and the melt temperature, i.e., the temperature value from the melt peak is plotted on a two dimensional scatter plot (block 104). In an alternative embodiment, the signal value from the melt peak is plotted along with the width value or the peak value and the temperature value in a three dimensional scatter plot (block 104). The data points on the scatter plots are then analyzed (block 106) as discussed below.

In another embodiment, a Gaussian probability function is fit to the first negative derivative data and the AUC is calculated for the Gaussian probability function. This technique is particularly useful to discriminate data wherein there are multiple peaks contained in the data, such as HRM data obtained from a heterogeneous mixture of double-stranded nucleic acids. In this circumstance, the total AUC will include the AUC from each of the multiple peaks. Thus, the total AUC from a heterogeneous mixture of double-stranded nucleic acids will be greater than the AUC for data from samples having a single peak.

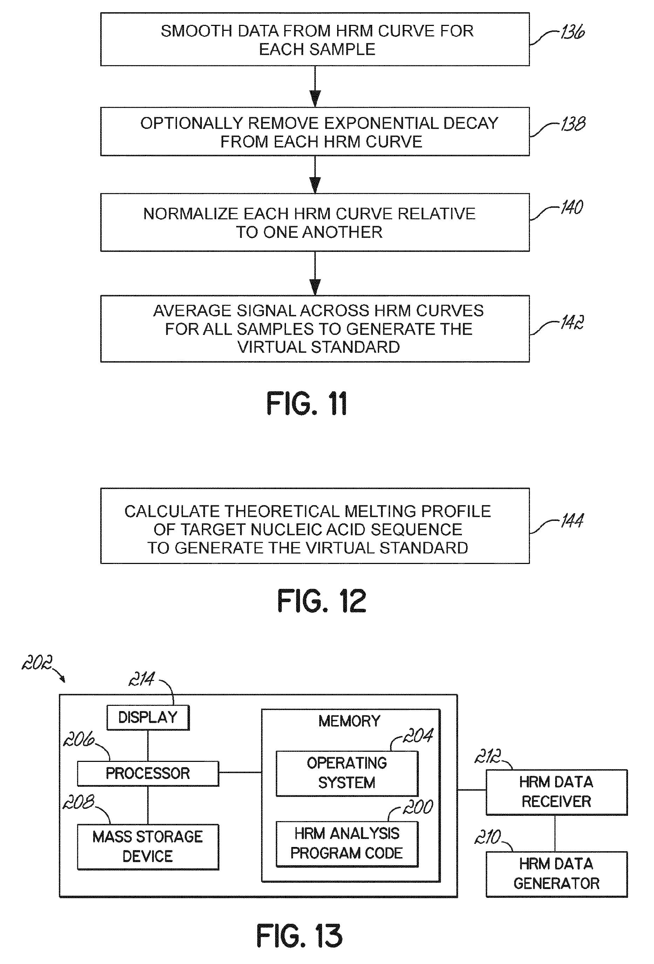

Regardless of how the data points are identified in the embodiments described herein, the data points 107 are plotted on a scatter plot for analysis (FIG. 1D) and the scatter plot may be displayed on a display (214 of FIG. 13). The data points 107 may be plotted on a scatter plot by a computer or other processing system. The scatter plot has a first axis that corresponds to the melt temperature value and at least a second axis that corresponds to one of the peak height value, peak width value, and area under the curve value. In another embodiment, the scatter plot has a third axis that differs from the second axis and corresponds to one of the peak height value, peak width value, and area under the curve value. The data point 107 is plotted as an individual point and multiple data points may form clusters. In some circumstances, different clusters of melt peak data points will be apparent. Alternatively, the clusters of melt peak data points may be further analyzed using cluster grouping algorithms that are capable of distinguishing between different clusters of data points. Cluster algorithm analysis is particularly useful for identifying which clusters to which each melt peak data point belongs or to identify clusters that are not otherwise apparent. Exemplary cluster analysis algorithms include circle analysis algorithms for two-dimensional scatter plots and sphere analysis algorithms for three-dimensional scatter plots. Cluster analysis algorithms allow for much more reliable automated calling of data points than is provided by traditional difference plot analysis.

In one embodiment, each melt peak data point on the scatter plot is compared with one or more standards. The standards include known values for one or more target sequences, such as the known values such as temperature value, height value, width value and/or area under the curve for each genotype of a heterogeneous gene. The Euclidian distance between the melt peak data point and the one or more standards is calculated. For samples resulting in the identification of two or more melt peaks, the distances calculated between first identified melt peak data point and the standard value is weighted more heavily than the distances calculated for each subsequent melt peak data point. For example, if a sample yields an HRM curve that results in three melt peaks being identified from the first negative derivative plot, the distance between the first melt peak data point and a standard value receives more weight during the subsequent analysis than the distances calculated for the second melt peak data point, which is subsequently weight more heavily than the distances calculated for the third melt peak data point. In one embodiment, a multiplier is used to weight the first peak more heavily than subsequent peaks. For example, the first peak may be multiplied by a value of four whereas the subsequent melt peaks are multiplied by multipliers decreasing in value, such as three, two, or one. For each data point, a ratio of the weighted distances between the closest standard and the second closest standard may be calculated. The ratio is used to indicate confidence with which a sample may be called as being like one of the first or the second closest standard. The ratio may be converted to a percentage efficiency between 100 percent and about 50 percent to indicate from the standard is calculated.

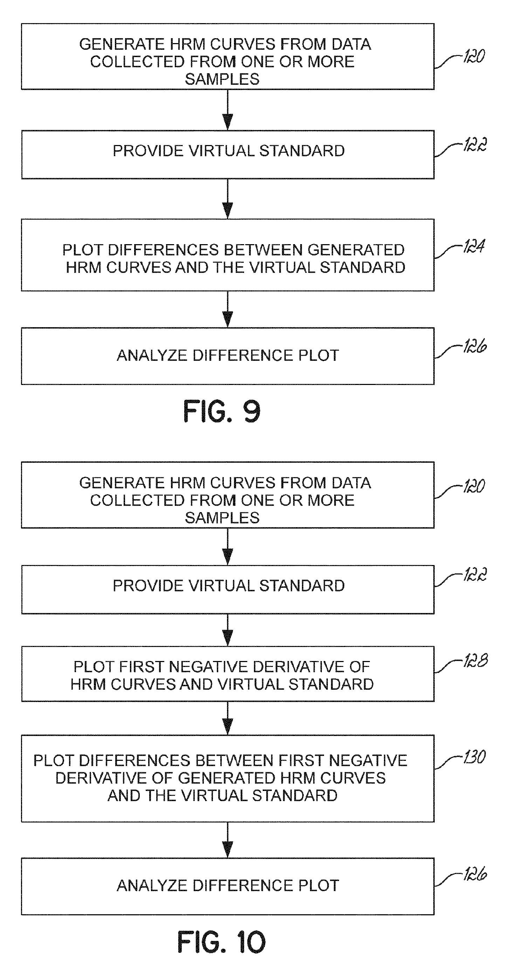

Another aspect of the invention is directed to visualizing HRM data utilizing a virtual standard 110 (FIG. 8A). For this aspect, HRM curves 112, 114, 116 (FIG. 8A) are generated from HRM data for each sample (FIG. 9, block 120). A virtual standard 110 is provided as a baseline (block 122). The virtual standard 110 is subtracted from the HRM curves 112, 114, 116 and the difference is plotted for each sample (block 124) and FIG. 8B. Alterations in the virtual standard can result in the visualization of greater differences between the data curves 112, 112', 114, 114', 116, 116' as illustrated in FIG. 8B.

In an alternative embodiment, the first negative derivative is plotted for the virtual standard and the HRM curves (FIG. 10, block 128) and the difference between the first negative derivatives is plotted (block 130) as a difference plot, and analyzed (block 126).

As mentioned above, the virtual standard contrasts with methods in routine use wherein an HRM curve from one sample in an experiment is used as a baseline against which other samples in the experiment are compared. Embodiments of the virtual standard are useful when comparing HRM data across experiments and even across different platforms, which contrasts with the routine methods of analyzing HRM data which do not allow for such comparisons.

The virtual standard may be generated using a number of techniques as described in the various embodiments below. In each of the virtual standard curve embodiments, the resulting virtual standard curve can then be saved and recalled for use with other data sets.

In one embodiment illustrated in FIG. 11, the raw HRM data from two or more samples are smoothed (block 136), the exponential decay is optionally removed (block 138), and the HRM data for the samples are normalized relative to one another (block 140). Then, the signal values that correspond to a temperature values for the normalized HRM curves across all of the samples are averaged to generate the virtual standard (block 142). The virtual standard can be used to generate a difference plot between each of the samples as described above.

In another embodiment, the virtual standard is a theoretical standardized curve based on the theoretical melting profile of the nucleic acid sequences being analyzed. The theoretical melting profile of a nucleic acid sequence can be based on the known parameters affecting the melt temperature of a double-stranded nucleic acid, such as the percent of each type of nucleic acid, the sequence of the nucleic acids and the length of the nucleic acid strand. For example, Visual OMP™ Nucleic Acid software from DNA Software could be used to generate a theoretical melt profile for a sequence of nucleic acids.

In an alternative embodiment, the virtual standards are user defined using a mathematical equation. This is done by allowing the user to define where the exponential region starts or stops, a maximum slope, the inclusion of desired inflection points and combinations thereof for an equation describing a theoretical standard curve. For example, in an embodiment, the user may generate a virtual standard by modifying an ideal melt curve defined by the Formula 1 below by setting the maximum signal value ($RFU_{max}$), the minimum signal value ($RFU_{min}$) and the melting temperature ($T_m$), wherein C is the curve parameter describing the steepness of the signal change and $T_i$ is the x-axis value.

$$\text{FORMULA 1:} \qquad RFU = RFU_{min} + RFU_{max}(1 - 1/(1 + e^{(C(Tm-Ti))}))$$

In an another embodiment, the user may generate a virtual standard by combining two weighted, ideal melt curves, such as two ideal curves generated by Formula 1 above. The combined curve is defined by Formula 2 below. In this embodiment, the user may set the maximum signal value ($RFU_{max}$), the minimum signal value ($RFU_{min}$), the melting temperatures for each curve ($Tm_1$ and $Tm_2$) and the steepness of the signal change for each curve ($C_1$ and $C_2$) and the weight given to each curve ($W_1$ and $W_2$).

$$\text{FORMULA 2:} \qquad RFU = RFU_{min} + RFU_{max}(1 - W_1/(1 + e^{(C_1(Tm1-Ti))}) - w2/(1 + e^{(C_2(Tm_2-Ti))}))$$

Virtual curves based on other modifications to ideal curves may be used as well.

In an alternative embodiment, a spline curve in a sigmoidal shape similar to a typical melt curve is provided that the user then forms into a desired shape such as by using a computer interface, such as a mouse, to click control the shape of the spline.

The user controlled virtual curves allow the user to match details from one or more of the sample HRM curves. By making portions of the virtual standard match the details seen on one or more of the HRM curves generated from the HRM data, while other portions of the virtual standard match portions of other HRM curves, the differences between the HRM curves can be made more obvious than simply picking one curve as a standard could, as was previously done. By allowing the user or the machine to adjust the shape of the virtual standard, some HRM curves in a data set may peak in the difference plot at one temperature, while the peak for other HRM curves would be at a different temperature or be in the negative direction from the first. Additionally, HRM curves that are distinct from those used to construct the virtual standard would exhibit different shapes distinct from the others.

The analytical processes of the invention may be embodied as a method, a computer program product that includes program code 200 to execute the method, and/or a computer system 202 configured to execute the method FIG. 13. The method includes the steps described herein and illustrated in FIGS. 1 to 12 for analyzing HRM data of a nucleic acid.

The program code 200 includes instructions executable on a computer system for carrying out the steps of the method. In one embodiment, the program code 200 includes instructions for analyzing HRM data and in particular, code for generating and displaying on a display 214 a scatter plot that includes the data points associated with the melting temperature. The scatter plot has a first axis for plotting the melt value for each data point and a second axis for plotting one of a peak value, a width value, or an area under the curve value for each data point. In another embodiment, the scatter plot has a third axis for plotting a third value for each data point that is the value remaining from the peak value, the width value, and the area under the curve value. Embodiments of the invention, whether implemented as part of an operating system 204, application, component, program code 200, object, module or sequence of instructions executed by one or more processing units 206 are referred to herein as "program code." The program code 200 typically comprises one or more instructions that are resident at various times in various memory 202 and storage devices 208 in the computer system 200 that, when read and executed by one or more processors 204 thereof cause that computer system 200 to perform the steps necessary to execute the instructions embodied in the program code 200 embodying the various aspects of the invention.

While embodiments of the invention are described in the context of fully functioning computing systems 200, those skilled in the art will appreciate that the various embodiments of the invention are capable of being distributed as a program product on a computer readable storage medium. The program product may embody a variety of forms. The invention applies equally regardless of the particular type of computer readable storage medium used to actually carry out the distribution of the program code 200. Examples of appropriate computer readable storage media for the program product include, but are not limited to, non-transitory recordable type media such as volatile and nonvolatile memory devices, floppy and other removable disks, hard disk drives, USB drives, optical disks (e.g. CD-ROM's, DVD's, Blu-Ray discs, etc.), among others.

Any of the individual processes described above or illustrated in FIGS. 1-12 may be formed into routines, procedures, methods, modules, objects, and the like, as is well known in the art. It should be appreciated that embodiments of the invention are not limited to the specific organization and allocation of program functionality described herein.

In addition, the systems for analyzing HRM data may further include a module for collecting the HRM data (i.e. a HRM data generator) 210 and a module for receiving HRM data 212. The HRM data collection module may include a thermal cycler and a device for detecting the signal value, that result from HRM analysis, such as a change in fluorescence from double-stranded nucleic acid over a range of temperatures. HRM data collection modules as known in the art may be used in accordance with the invention. The HRM data receiving module includes components and/or program code to receive HRM data from the HRM data collection module.

Example 1

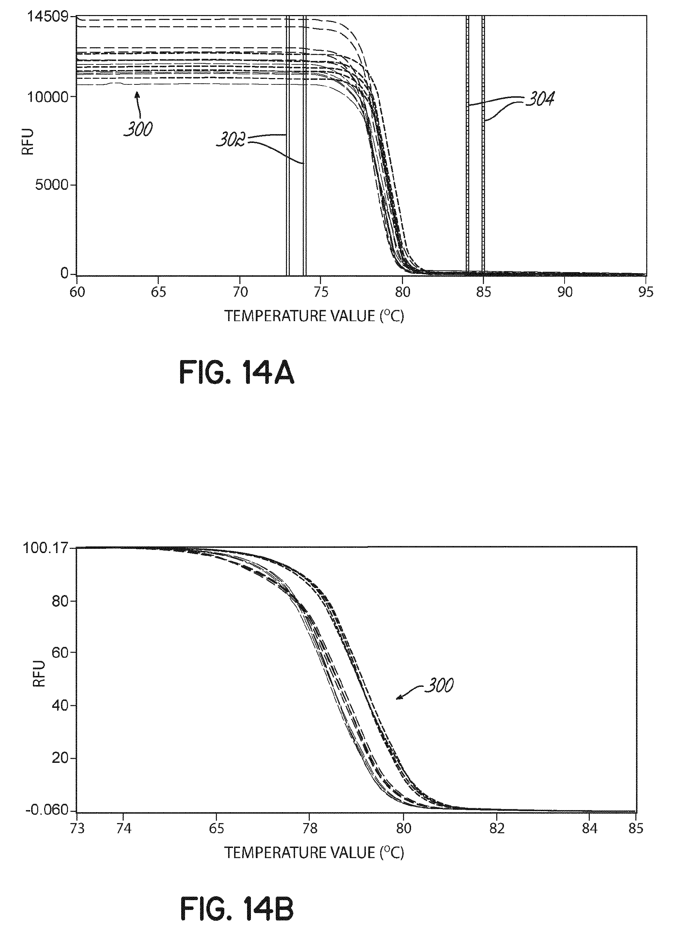

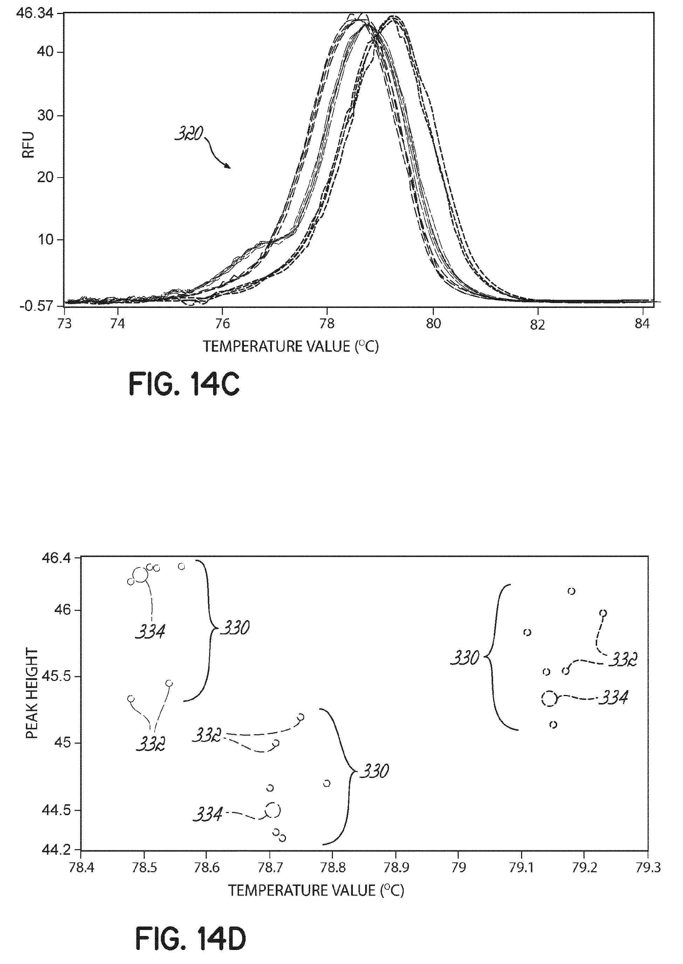

A set of samples representing two homogenous samples types and one heterogeneous mix of the two was run in a PikoReal instrument and analyzed by the melt peak method. For this example, olfactory receptor, family 10, subfamily J, member 5 (OR10J5) amplicons having a single nucleotide polymorphism (G or A) identified as rs4656837 were amplified using a standard PCR protocol in the presence of SYBR Green. After amplification, the samples were slowly heated and fluorescence measured at regular intervals. As shown in FIG. 14A, HRM curves 300 were generated for 18 samples and the exponential decay removed. The HRM curves 300 were normalized based on user identified areas of the saturation region 302 and background region 304. FIG. 14B illustrates the normalized HRM curves 304. FIG 14C illustrates the first negative derivative plots 320 from the normalized HRM curves of FIG. 14B. FIG. 14D illustrates a scatter plot of the melt peak data points identified from the first negative derivative plots of FIG. 14C. Clusters 330 of data points 332 are observed near each of the three standards 334 (larger circles, S1, S2, and S3 from left to right).

These data indicate that the samples include three different mixtures of double-stranded nucleic acids. The first group of samples is homozygous for an amplicon having the G SNP. The second group of sample is homozygous for an amplicon having the A SNP. The third group is a heterozygous mixture of the A and G SNP amplicons. As shown in Table 1, below, the analytical methods described herein clearly distinguish the difference between the homozygous and heterozygous clusters of data points. Samples 1-6 clustered nearest to standard S1 in the upper left hand quadrant of the scatter plot. Samples 7-12 clustered to standard S2 in the central lower region of the scatter plot and samples 13-18 clustered to standard S3 in the upper right region of the scatter plot. By plotting the peak heights on the scatter plot as a function of the melt temperature, the clusters are easily further analyzed such as by determining their Euclidean distance from the first and second closest standards, and calculating the ratio of these distances. The ratio was then used to calculate the percentage efficiency for the sample.

Table 1

| Sample No. | Melt Temp. | Peak Height | Dist. to closest stand. | Dist to 2d closest stand. | Ratio of D1/D2 | Percentage Efficiency |
|---|---|---|---|---|---|---|
| 1 | 78.48 | 46.22 | 1 (S1) | 111 (S2) | 0.01 | 99.9% |
| 2 | 78.51 | 46.33 | 1.5 (S1) | 112 (S2) | 0.01 | 98.7% |
| 3 | 78.52 | 46.32 | 4 (S1) | 110 (S2) | 0.04 | 96% |
| 4 | 78.57 | 46.34 | 15 (S1) | 107 (52) | 0.14 | 96.5% |
| 5 | 78.48 | 45.34 | 49 (S1) | 72 (52) | 0.68 | 59.5 |
| 6 | 78.54 | 45.46 | 44 (S1) | 65 (S2) | 0.68 | 59.6 |
| 7 | 78.70 | 44.72 | 6 S2) | 101 (S1) | 0.06 | 94.4 |
| 8 | 78.71 | 45.02 | 15 (S2) | 87 (S1) | 0.17 | 85.3 |
| 9 | 78.71 | 44.34 | 11 (S2) | 119 (S1) | 0.09 | 91.5 |
| 10 | 78.72 | 44.28 | 9 (S2) | 123 (S1) | 0.07 | 93.2 |
| 11 | 78.75 | 45.22 | 38 (S2) | 87 (S1) | 0.44 | 69.6 |
| 12 | 78.79 | 44.72 | 22 (S2) | 102 (S3) | 0.22 | 82.3 |
| 13 | 79.11 | 45.82 | 26 (S3) | 132 (S2) | 0.20 | 83.5 |
| 14 | 79.74 | 45.53 | 8 (S3) | 125 (S2) | 0.06 | 94 |
| 15 | 79.75 | 45.13 | 8 (S3) | 119 (S2) | 0.07 | 93.8 |
| 16 | 79.77 | 45.56 | 10 (S3) | 133 (S2) | 0.08 | 93 |

(continued)

| Sample No. | Melt Temp. | Peak Height | Dist. to closest stand. | Dist to 2d closest stand. | Ratio of D1/D2 | Percentage Efficiency |
|---|---|---|---|---|---|---|
| 17 | 79.78 | 46.14 | 43 (S3) | 152 (S2) | 0.28 | 98 |
| 18 | 79.23 | 46.00 | 39 (S3) | 158 (S2) | 0.25 | 80.2 |

Example 2

In this example, the peak width was plotted to further discriminate the signal values of the HRM melt curves. When mixed populations of double-stranded nucleic acids were present in the same sample undergoing HRM analysis, they tended to make the resulting HRM curve wider, especially when the difference in the melting temperatures of the individual probes were greater. As observed with the data presented below, this method was found to be particularly useful when analyzing multiplexed reactions.

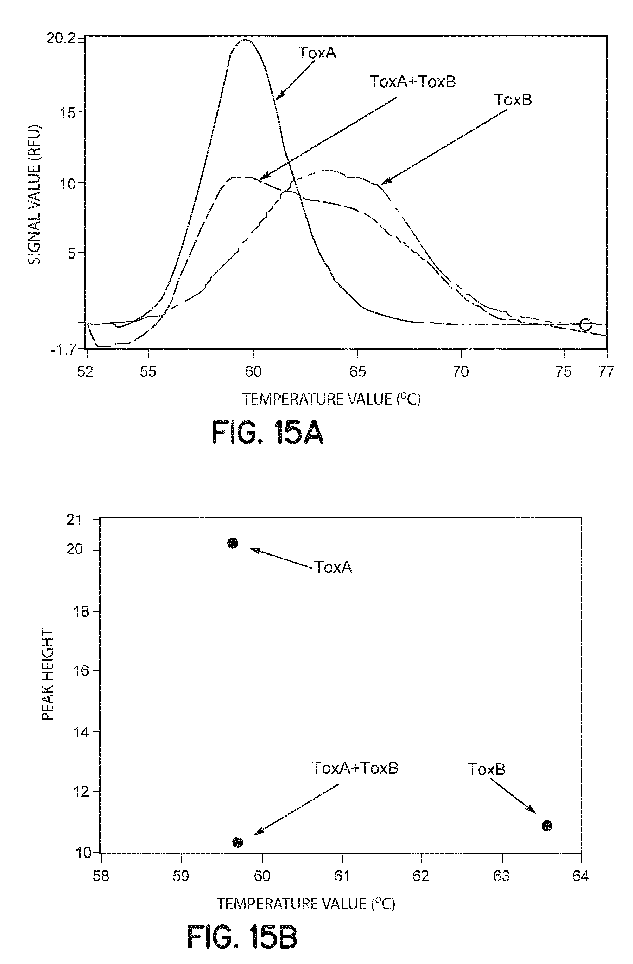

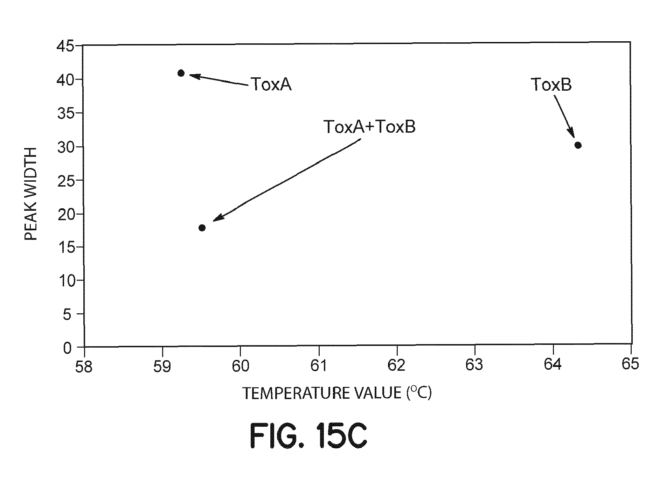

For this example, ToxA and ToxB were amplified using a standard PCR protocol in the presence two Solaris primers and fluorescent probes for ToxA and ToxB in c. *difficile.* FIG. 15A shows data from a qPCR and HRM analysis experiment wherein each probe was used by itself or in combination with the other probe. The data demonstrate that while the ToxA+ToxB curve had a similar melting temperature to the ToxA probe by itself, it is easily distinguished by the peak height (FIG. 15B) and the peak width (FIG. 15C).

The following numbered clauses describe some embodiments of the invention, while the scope of protection is defined by the appended claims.

Clause 1: A method of analyzing high resolution melt (HRM) data wherein the HRM data is characterized by a plurality of data points each including a signal value associated with the concentration of a double-stranded nucleic acid in a sample and a temperature value associated with a the temperature of the sample, the method comprising:

generating a HRM curve from the HRM data;
plotting the first negative derivative of the HRM curve;
detecting the melt peak of the first negative derivative plot;
plotting a data point associated with the melt peak on a scatter plot; and
analyzing the data point on the scatter plot.

Clause 2: The method of clause 1 wherein generating the HRM curve from the HRM data comprises at least one of smoothing the HRM data or removing exponential decay from the HRM data.

Clause 3: The method of clause 1 or 2 wherein the melt peak is a data point along the first negative derivative plot having the greatest amplitude.

Clause 4: The method of clause 1 or 2 further comprising fitting a Gaussian probability function to the first negative derivative plot; and
the melt peak is a data point along the Gaussian probability function having the greatest amplitude.

Clause 5: The method of clause 4 further comprising subtracting the Gaussian probability function from the first negative derivative plot and identifying a second melt peak from the subtracted data set;
plotting a data point associated with the second melt peak on the scatter plot; and
analyzing the data points on the scatter plot.

Clause 6: The method of clause 5 wherein the second melt peak is identified as data point along the subtracted data set having the greatest amplitude.

Clause 7: The method of clause 5 further comprising fitting a second Gaussian probability function to the subtracted data set; and
the second melt peak is a data point along the second Gaussian probability function having the greatest amplitude.

Clause 8: The method of clause 6 further comprising subtracting the second Gaussian probability function from the first subtracted data set to form a second subtracted data set and identifying a third melt peak from the second subtracted data set;
plotting a data point associated with the third melt peak on the scatter plot; and

analyzing the data points on the scatter plot.

Clause 9: The method of clause 8 further comprising fitting a third Gaussian probability function to the second subtracted data set; and
the third melt peak is a data point along the second subtracted data set having the greatest amplitude.

Clause 10: A method of analyzing HRM data from each of a first sample and a second sample, wherein the HRM data are characterized by a plurality of data points each including a signal value associated with the concentration of a double-stranded nucleic acid in each sample and a temperature value associated with a the temperature of each sample, the method comprising:

generating a HRM curve from the HRM data for each sample;
plotting the first negative derivative of the HRM curve for each sample;
detecting the melt peak of the first negative derivative plot for each sample;
plotting a data point associated with the melt peak for each sample on a scatter plot; and
analyzing the data points.

Clause 11: The method of clause 10 wherein generating the HRM curve from the HRM data for each sample comprises at least one of smoothing the HRM data for each sample or removing exponential decay from the HRM data for each sample.

Clause 12: The method of clause 10 or 11 further comprising normalizing the HRM curves for each sample relative to one another.

Clause 13: The method of one of clauses 11, 12, or 13 wherein the melt peak is a data point along the first negative derivative plot for each sample having the greatest amplitude.

Clause 14: The method of one of clauses 11, 12, or 13 further comprising fitting a Gaussian probability function to the first negative derivative plot for each sample; and
the melt peak for each sample is a data point along the Gaussian probability function for the sample having the greatest amplitude.

Clause 15: The method of clause 14 further comprising subtracting the Gaussian probability function from the first negative derivative plot for each sample and identifying a second melt peak from the subtracted data set for each sample;
plotting a data point associated with the second melt peak on the scatter plot; and
analyzing the data points on the scatter plot.

Clause 16: The method of clause 15 wherein the second melt peak is identified as a data point along the subtracted data set for each sample having the greatest amplitude.

Clause 17: The method of clause 16 further comprising fitting a second Gaussian probability function to the subtracted data set for each sample; and
the second melt peak for each sample is a data point along the subtracted data set having the greatest amplitude.

Clause 18: The method of clause 16 further comprising subtracting the second Gaussian probability function for each sample from the first subtracted data set for each sample to form a second subtracted data set and identifying a third melt peak for each sample from the second subtracted data set;
plotting a data point associated with the third melt peak on the scatter plot; and
analyzing the data points on the scatter plot.

Clause 19: The method of clause 18 further comprising fitting a third Gaussian probability function to the second subtracted data set for each sample; and
the third melt peak for each sample is a data point along the second subtracted data set having the greatest amplitude.

Clause 20: A method of analyzing HRM data for each of one or more samples wherein the HRM data is characterized by a plurality of data points each including a signal value associated with the concentration of a double-stranded nucleic acid in a sample and a temperature value associated with a the temperature of the sample, the method comprising:

generating a HRM curve from the HRM data for each of the one or more samples;
normalizing the each HRM curve relative to one another;
plotting the first negative derivative of each normalized HRM curve;
identifying the melt peak of the first negative derivative plot for each sample wherein the melt peak has a temperature value and a peak height value;
calculating at least one of a width value or an area under the curve value wherein the width value or area under the curve value is calculated or measured for each first negative derivative plot at a fraction of the melt peak height value;
plotting a data point for each of the one or more samples, the data point having a temperature value and at least one of the width value, a peak height value or an area under the curve value; and
analyzing the data points.

Clause 21: The method of clause 20 wherein generating the HRM curve from the HRM data for each of one or more samples comprises at least one of smoothing the HRM data or removing exponential decay from the HRM data.

Clause 22: The method of clause 20 or 21 wherein the one of the width value or the area under the curve value is calculated at about 50 percent of the peak height.

Clause 23: The method of clause 20 or 21 wherein the one of the width value or the area under the curve value is calculated at a fraction of the peak height value that is in the range between about 15 percent of the peak height value and about 85 percent of the peak height value.

Clause 24: The method of one of the preceding clauses further comprising performing a cluster analysis on the data points on the scatter plot.

Clause 25: The method of one of the preceding clauses further comprising plotting at least one standard having melt values for a known double-stranded nucleic acid on the scatter plot, wherein the melt values includes a temperature value and at least one of a peak height value, a width value or an area under the curve value; and calculating the Euclidian distance from each data point to the at least one standard.

Clause 26: The method of clause 25 further comprising plotting at least a second standard having melt values for a second known double-stranded nucleic acid on the scatter plot; and calculating the distance from each data point to the second standard.

Clause 27: The method of clause 26 further comprising identifying the standard that is closest to each data point.

Clause 28: The method of clause 27 further comprising calculating a ratio of the distances between the standard that is closest to the data point and the standard that is second closest to the data point.

Clause 29: A method of visualizing HRM data from one or more samples wherein the HRM data is characterized by a plurality of data points each including a signal value associated with the concentration of a double-stranded nucleic acid in a sample and a temperature value associated with a the temperature of the sample, the method comprising:

generating a HRM curve from the HRM data for each sample;
providing a virtual standard; and
plotting the differences between the HRM curve for each sample and the virtual curve.

Clause 30: A method of visualizing HRM data from one or more samples wherein the HRM data is characterized by a plurality of data points each including a signal value associated with the concentration of a double-stranded nucleic acid in a sample and a temperature value associated with a the temperature of the sample, the method comprising:

generating a HRM curve from the HRM data for each sample;
providing a virtual standard;
plotting the first negative derivative of the HRM curve for each sample and

plotting the differences between the first negative derivative plot for each sample and the virtual curve.

Clause 31: The method of clause 29 or 30 wherein the virtual standard comprises:

averaging the signal across the HRM curves for each sample to result in a virtual standard curve.

Clause 32: The method of clause 31 further comprising, prior to averaging the signal values across the HRM curves for each sample, at least one of smoothing the HRM for each sample, removing exponential decay from the HRM curve for each sample; and normalizing the HRM curves for each sample to one another.

Clause 33: The method of clause 29 or 30 wherein the virtual standard comprises calculating a theoretical melting profile of a target double-stranded nucleic acid to generate the virtual standard.

Clause 34: The method of clause 29 or 30 wherein the virtual standard comprises providing a formula to calculate the virtual standard wherein the variables in the formula are adjustable to define end points of the exponential region, a maximum slope for the exponential region, inclusion of desired inflection points in the exponential region, and combinations thereof.

Clause 35: The method of clause 29 or 30 wherein the providing the virtual standard comprises providing a spline curve in a sigmoidal shape, wherein the shape of the spline curve may be altered by the user using a computer interface.

While the present invention has been illustrated by the description of specific embodiments thereof, and while the embodiments have been described in considerable detail, it is not intended to restrict or in any way limit the scope of the appended claims to such detail. The various features discussed herein may be used alone or in any combination. Additional advantages and modifications will readily appear to those skilled in the art. The invention in its broader aspects is therefore not limited to the specific details, representative apparatus and methods and illustrative examples shown and described. Accordingly, departures may be made from such details without departing from the scope or spirit of the general inventive concept.

## Claims

1.  A method of analyzing high resolution melt (HRM) data from a first sample wherein the HRM data is **characterized by** a plurality of data points each including a signal value associated with the concentration of a double-stranded nucleic acid in a sample and a temperature value associated with a the temperature of the sample, the method comprising:

    generating a HRM curve from the HRM data for the first sample;
    plotting the first negative derivative of the HRM curve for the first sample;
    detecting the melt peak of the first negative derivative plot for the first sample;
    plotting a data point associated with the melt peak for the first sample on a scatter plot; and
    analyzing the data point on the scatter plot.

2.  The method of claim 1 wherein generating the HRM curve from the HRM data comprises at least one of smoothing the HRM data or removing exponential decay from the HRM data.

3.  The method of claim 1 wherein the melt peak is a data point along the first negative derivative plot having the greatest amplitude.

4.  The method of claim 1 further comprising fitting a Gaussian probability function to the first negative derivative plot; and the melt peak is a data point along the Gaussian probability function having the greatest amplitude.

5.  The method of claim 4 further comprising subtracting the Gaussian probability function from the first negative derivative plot and identifying a second melt peak from the subtracted data set;
    plotting a data point associated with the second melt peak on the scatter plot; and
    analyzing the data points on the scatter plot.

6. The method of claim 5 wherein the second melt peak is identified as data point along the subtracted data set having the greatest amplitude.

7. The method of claim 5 further comprising fitting a second Gaussian probability function to the subtracted data set; and the second melt peak is a data point along the second Gaussian probability function having the greatest amplitude.

8. The method of claim 7 further comprising subtracting the second Gaussian probability function from the first subtracted data set to form a second subtracted data set and identifying a third melt peak from the second subtracted data set;
plotting a data point associated with the third melt peak on the scatter plot; and
analyzing the data points on the scatter plot.

9. The method of claim 8 further comprising fitting a third Gaussian probability function to the second subtracted data set; and
the third melt peak is a data point along the second subtracted data set having the greatest amplitude.

10. A method of analyzing HRM data for each of one or more samples wherein the HRM data is **characterized by** a plurality of data points each including a signal value associated with the concentration of a double-stranded nucleic acid in a sample and a temperature value associated with a the temperature of the sample, the method comprising:

generating a HRM curve from the HRM data for each of the one or more samples;
normalizing the each HRM curve relative to one another;
plotting the first negative derivative of each normalized HRM curve;
identifying the melt peak of the first negative derivative plot for each sample wherein the melt peak has a temperature value and a peak height value;
calculating at least one of a width value or an area under the curve value wherein the width value or area under the curve value is calculated or measured for each first negative derivative plot at a fraction of the melt peak height value;
plotting a data point for each of the one or more samples, the data point having a temperature value and at least one of the width value, a peak height value or an area under the curve value; and
analyzing the data points.

11. The method of claim 10 wherein generating the HRM curve from the HRM data for each of one or more samples comprises at least one of smoothing the HRM data or removing exponential decay from the HRM data.

12. The method of claim 10 or 11 wherein the one of the width value or the area under the curve value is calculated at a fraction of the peak height value that is in the range between about 15 percent of the peak height value and about 85 percent of the peak height value.

13. The method of one of the preceding claims further comprising performing a cluster analysis on the data points on the scatter plot.

14. The method of one of the preceding claims further comprising plotting at least one standard having melt values for a known double-stranded nucleic acid on the scatter plot, wherein the melt values includes a temperature value and at least one of a peak height value, a width value or an area under the curve value; and
calculating the Euclidian distance from each data point to the at least one standard.

15. The method of claim 14 further comprising plotting at least a second standard having melt values for a second known double-stranded nucleic acid on the scatter plot;
calculating the distance from each data point to the second standard, and
identifying the standard that is closest to each data point.

16. The method of claim 15 further comprising calculating a ratio of the distances between the standard that is closest to the data point and the standard that is second closest to the data point.

17. A method of visualizing HRM data from one or more samples wherein the HRM data is **characterized by** a plurality of data points each including a signal value associated with the concentration of a double-stranded nucleic acid in a sample and a temperature value associated with a the temperature of the sample, the method comprising:

generating a HRM curve from the HRM data for each sample;
providing a virtual standard; and
plotting the differences between the HRM curve for each sample and the virtual curve.

18. (Original) A method of visualizing HRM data from one or more samples wherein the HRM data is **characterized by** a plurality of data points each including a signal value associated with the concentration of a double-stranded nucleic acid in a sample and a temperature value associated with a the temperature of the sample, the method comprising:

generating a HRM curve from the HRM data for each sample;
providing a virtual standard;
plotting the first negative derivative of the HRM curve for each sample and
plotting the differences between the first negative derivative plot for each sample and the virtual curve.

19. The method of claim 17 or 18 wherein the virtual standard comprises:

averaging the signal across the HRM curves for each sample to result in a virtual standard curve.

20. The method of claim 19 further comprising, prior to averaging the signal values across the HRM curves for each sample, at least one of smoothing the HRM for each sample, removing exponential decay from the HRM curve for each sample; and normalizing the HRM curves for each sample to one another.

21. The method of claim 17 or 18 wherein the virtual standard comprises calculating a theoretical melting profile of a target double-stranded nucleic acid to generate the virtual standard.

22. The method of claim 17 or 18 wherein the virtual standard comprises providing a formula to calculate the virtual standard wherein the variables in the formula are adjustable to define end points of the exponential region, a maximum slope for the exponential region, inclusion of desired inflection points in the exponential region, and combinations thereof.

23. The method of claim 17 or 18 wherein the providing the virtual standard comprises providing a spline curve in a sigmoidal shape, wherein the shape of the spline curve may be altered by the user using a computer interface.

FIG. 1A

FIG. 1B

FIG. 1C

FIG. 1D

20 — GENERATE HRM CURVE

22 — PLOT FIRST NEGATIVE DERIVATIVE
OF HRM CURVE

24 — IDENTIFY MELT PEAK

26 — ANALYZE MELT PEAK

FIG. 2

28 — PLOT HRM CURVE WITH DATA
POINTS FROM HRM ANALYSIS

30 — SMOOTH CURVE

32 — REMOVE EXPONENTIAL
DECAY

FIG. 3

GENERATE HRM CURVE — 20

PLOT FIRST NEGATIVE DERIVATIVE OF HRM CURVE — 22

FIT GAUSSIAN PROBABILITY FUNCTION TO FIRST NEGATIVE DERIVITIVE PLOT — 54

IDENTIFY PEAK GAUSSIAN PROBABILITY FUNCTION AS MELT PEAK DATA POINT — 56

ANALYZE MELT PEAK DATA POINTS — 58

FIG. 4

GENERATE HRM CURVE FOR EACH SAMPLE — 60

NORMALIZE HRM CURVES TO ONE ANOTHER — 62

PLOT FIRST NEGATIVE DERIVATIVE
FOR EACH NORMALIZED OF HRM CURVE — 64

IDENTIFY MELT PEAKS FOR EACH SAMPLE — 66

PLOT MELT PEAK DATA POINTS FOR
EACH SAMPLE ON SCATTER PLOT — 68

ANALYZE MELT PEAK DATA
POINTS ON SCATTER PLOT — 70

# FIG. 5

20 — GENERATE HRM CURVE

22 — PLOT FIRST NEGATIVE DERIVATIVE
OF HRM CURVE

76 — FIT FIRSTGAUSSIAN PROBABILITY FUNCTION TO
FIRST NEGATIVE DERIVITIVE CURVE

78 — IDENTIFY THE PEAK OF GAUSSIAN
PROBABILITY FUNCTION AS A DATA
POINT REPRESENTING A MELT PEAK

SUBTRACT GAUSSIAN PROBABLITY
FUNCTION FROM FIRST NEGATIVE
DERIVATIVE CURVE — 80

FIT SECOND GAUSSIAN PROBABILITY
FUNCTION TO SUBTRACTED DATA — 84

DETECT THE PEAK OF GAUSSIAN
PROBABILITY FUNCTION AS A DATA
POINT REPRESENTING A MELT PEAK — 86

Y — MELT PEAK DETECTED? — 88

N

ANALYZE MELT PEAKS — 90

FIG. 6

GENERATE A HRM CURVE FOR EACH SAMPLE — 94

NORMALIZE HRM CURVES TO ONE ANOTHER — 96

PLOT FIRST NEGATIVE DERIVATIVE FOR EACH NORMALIZED HRM CURVE — 99

IDENTIFY MELT PEAK FOR EACH SAMPLE — 100

IDENTIFY WIDTH OF EACH FIRST NEGATIVE DERIVATIVE HRM CURVE AT A SPECIFIED FRACTION OF THE MELT PEAK HEIGHT — 102

PLOT DATAPOINT HAVING A TEMPERATURE VALUE AND AT LEAST ONE OF A WIDTH VALUE OR A PEAK HEIGHT VALUE OR AN AUC VALUE ON A SCATTER PLOT — 104

ANALYZE DATA POINTS ON SCATTER PLOT — 106

FIG. 7

FIG. 8A

FIG. 8B

GENERATE HRM CURVES FROM DATA COLLECTED FROM ONE OR MORE SAMPLES — 120

↓

PROVIDE VIRTUAL STANDARD — 122

↓

PLOT DIFFERENCES BETWEEN GENERATED HRM CURVES AND THE VIRTUAL STANDARD — 124

↓

ANALYZE DIFFERENCE PLOT — 126

**FIG. 9**

GENERATE HRM CURVES FROM DATA COLLECTED FROM ONE OR MORE SAMPLES — 120

↓

PROVIDE VIRTUAL STANDARD — 122

↓

PLOT FIRST NEGATIVE DERIVATIVE OF HRM CURVES AND VIRTUAL STANDARD — 128

↓

PLOT DIFFERENCES BETWEEN FIRST NEGATIVE DERIVATIVE OF GENERATED HRM CURVES AND THE VIRTUAL STANDARD — 130

↓

ANALYZE DIFFERENCE PLOT — 126

**FIG. 10**

```
┌─────────────────────────────────────────┐
│  SMOOTH DATA FROM HRM CURVE FOR          │──136
│           EACH SAMPLE                    │
└─────────────────────────────────────────┘
                    │
                    ▼
┌─────────────────────────────────────────┐
│  OPTIONALLY REMOVE EXPONENTIAL DECAY     │──138
│        FROM EACH HRM CURVE               │
└─────────────────────────────────────────┘
                    │
                    ▼
┌─────────────────────────────────────────┐
│  NORMALIZE EACH HRM CURVE RELATIVE       │──140
│          TO ONE ANOTHER                  │
└─────────────────────────────────────────┘
                    │
                    ▼
┌─────────────────────────────────────────┐
│  AVERAGE SIGNAL ACROSS HRM CURVES        │
│  FOR ALL SAMPLES TO GENERATE THE         │──142
│          VIRTUAL STANDARD                │
└─────────────────────────────────────────┘
```

## FIG. 11

```
┌─────────────────────────────────────────┐
│  CALCULATE THEORETICAL MELTING PROFILE   │
│   OF TARGET NUCLEIC ACID SEQUENCE        │──144
│   TO GENERATE THE VIRTUAL STANDARD       │
└─────────────────────────────────────────┘
```

## FIG. 12

202

## FIG. 13

FIG. 14A

FIG. 14B

FIG. 14C

FIG. 14D

FIG. 15A

FIG. 15B

FIG. 15C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61660581 B

- US 13799139 B